# EUROPEAN PATENT APPLICATION

(11) **EP 3 285 226 A1**
(43) Date of publication of application: **21.02.2018**
(21) Application number: 16796054.1
(22) Date of filing: 06.04.2016
(51) Int. Cl.: G06Q 50/24, G06F 13/00

(54) **TERMINAL DEVICE, SERVER DEVICE, DISPLAY CONTROL METHOD, AND DISPLAY CONTROL PROGRAM**

(30) Priority: 19.05.2015 JP 2015101912
(71) Applicant: Panasonic Healthcare Holdings Co., Ltd., Tokyo 105-8433 (JP)
(72) Inventor: MINEMURA, Atsushi, Ehime, 791-0395 (JP); OZAKI, Nobuhiko, Ehime, 791-0395 (JP)
(74) Representative: Grünecker Patent- und Rechtsanwälte PartG mbB
(86) International application number: PCT/JP2016/001931
(87) International publication number: WO 2016/185657

(57) **Abstract**

This terminal device (11) controls a display unit so as to: transmit a code inputted to a code input screen to a server device (12); receive, from the server device (12), screen information corresponding to a pharmacy specified on the basis of the code; and display a first service selection screen on the basis of the screen information. The first service selection screen accepts an operation by which the user of the terminal device (11) selects a desired service from among options that include common and exclusive services.

## Description

### Technical Field

The present invention relates to a terminal apparatus for controlling a display screen of application software, a server apparatus, a display control method, and a display control program.

### Background Art

Conventionally, application software that allows the use of services provided by pharmacies (hereinafter referred to as dedicated application) is known. The dedicated application operates in terminal apparatuses such as smartphones and tablets, for example. By use of the dedicated application, the patient can browse the track of medicine history information of taken medicinal products (for example, an electronic "okusuri-techou"), and can preliminarily transmit, to a pharmacy, data on medical prescriptions received from a hospital and the like. For example, PTL 1 discloses a technique that allows a patient to browse medical prescription data on a smartphone, and transmit the medical prescription data to a personal computer of a pharmacy.

In the case where dedicated applications which are different among pharmacies are provided, however, the following problems arise.

For example, inconveniently, users (patients) who usually use a plurality of pharmacies have to install and use the dedicated applications of respective pharmacies.

In addition, for example, there is a problem of the cost for developing and running the dedicated application. Consequently, while pharmacies having sufficient funding can develop and run a dedicated application, it is difficult for pharmacies which does not have sufficient funding to develop and run a dedicated application.

In addition, for example, pharmacies can facilitate the patients who come to the pharmacy to use the dedicated application to make the patients to continuously use the same pharmacy, but cannot facilitate potential patients (potential customers) to use the dedicated application, and consequently it is difficult win new patients.

### Citation List

### Patent Literature

PTL 1
Japanese Patent Application Laid-Open No. 2015-1944

### Summary of Invention

### Technical Problem

To solve the above-mentioned problems, it is conceivable to unify the platforms of services of pharmacies. To be more specific, it is conceivable to provide services to patients of pharmacies with one dedicated application so as to collectively lead many patients to use the dedicated application. It is conceivable that the dedicated application is run by the operating company of the dedicated application, and each pharmacy pays the fee of dedicated application to the operating company of the dedicated application.

With this configuration, the patients can avoid the inconvenience of using the different dedicated applications of respective pharmacies. Meanwhile, the pharmacies can reduce the cost of developing and running the dedicated application, and in addition, can make the patients to continuously use the same pharmacies while winning new patients.

In the case where services are provided to the patients of pharmacies with one dedicated application, however, a service common to pharmacies are provided with the dedicated application, and consequently, it is difficult to provide a service therefore makes a differentiation from other pharmacies.

An object of the present invention is to achieve provision of a service that makes a differentiation from other pharmacies in addition to a service common to pharmacies in the case where services are provided to the patients of pharmacies with one dedicated application.

### Solution to Problem

A terminal apparatus of an embodiment of the present invention is configured to for operating an application for providing a service of a plurality of pharmacies, the terminal apparatus including: a communication section that exchanges information with a server apparatus; a display section that displays the application; and a control section that controls the communication section and the display section, in which when an operation of identifying a pharmacy is received in the application, the control section controls the communication section to transmit information of the operation to the server apparatus, when the communication section receives screen information corresponding to a pharmacy identified by the server apparatus based on the information of the operation from the server apparatus, the control section generates a first service selection screen based on the screen information, and controls the display section to display the first service selection screen, when the operation is not received in the application, the control section controls a display section to display a second service selection screen, the first service selection screen is a screen that includes options of a common service common to the plurality of pharmacies, and options of a unique service unique to the pharmacy identified by the server apparatus, and receives an operation of a user of the terminal apparatus for selecting a desired service from among the options of the common service and the options of the unique service, and the second service selection screen is a screen that includes the options of the common service without including the options of the unique service, and receives an operation of the user of the terminal apparatus for selecting a desired service from among the options of the common service.

A server apparatus of an embodiment of the present invention includes: a communication section that exchanges information with a terminal apparatus, the terminal apparatus being configured to operate an application for providing a service of a plurality of pharmacies, and display a first service selection screen, the first service selection screen being configured to include options of a common service common to the plurality of pharmacies and options of a unique service unique to a pharmacy, and receive an operation of a user of the terminal apparatus for selecting a desired service from among the options of the common service and the options of the unique service; a storage section that stores an identification information management table in which screen identification information capable of identifying screen information of the application is linked for each pharmacy, and a screen information management table in which the screen information is linked for each screen identification information; and a control section that acquires, when an operation of identifying a pharmacy is received in the application of the terminal apparatus and information of the operation is received from the terminal apparatus, the screen identification information from the identification information management table based on the information of the operation, acquires the screen information from the screen information management table based on the screen identification information, and controls the communication section to transmit the screen information to the terminal apparatus; in which in the terminal apparatus, the screen information is used for generating a second service selection screen, the second service selection screen being configured to include options of a common service common to the plurality of pharmacies and options of a unique service unique to a pharmacy identified by the operation, and receive an operation of the user of the terminal apparatus for selecting a desired service from among the options of the common service and the options of the unique service.

A display control method of a terminal apparatus of an embodiment of the present invention is configured for operating an application for providing a service of a plurality of pharmacies, the method including: when an operation of identifying a pharmacy is received in the application, generating a first service selection screen based on screen information corresponding to a pharmacy identified based on information of the operation, and controlling a display section to display the first service selection screen; and when the operation is not received in the application, controlling the display section to display a second service selection screen, in which the first service selection screen is a screen that includes options of a common service common to the plurality of pharmacies, and options of a unique service unique to a pharmacy identified by a server apparatus, and receives an operation of a user of the terminal apparatus for selecting a desired service from among the options of the common service and the options of the unique service, and the second service selection screen is a screen that includes the options of the common service without including the options of the unique service, and receives an operation of the user of the terminal apparatus for selecting a desired service from among the options of the common service.

A display control program of an embodiment of the present invention is configured to be executed by a computer for operating an application for providing a service of a plurality of pharmacies, in which the program causes the computer to, when an operation of identifying a pharmacy is received in the application, generate a first service selection screen based on screen information corresponding to a pharmacy identified based on information of the operation, and control the display section to display the first service selection screen; the program causes the computer to, when the operation is not received in the application, control the display section to display a second service selection screen; the first service selection screen is a screen that includes options of a common service common to the plurality of pharmacies, and options of a unique service unique to a pharmacy identified by a server apparatus, and receives an operation of a user of the terminal apparatus for selecting a desired service from among the options of the common service and the options of the unique service; and the second service selection screen is a screen that includes the options of the common service without including the options of the unique service, and receives an operation of the user of the terminal apparatus for selecting a desired service from among the options of the common service.

### Advantageous Effects of Invention

According to the present invention, it is possible to achieve provision of a service that makes a differentiation from other pharmacies in addition to a service common to pharmacies in the case where services are provided to the patients of pharmacies with one dedicated application.

### Brief Description of Drawings

FIG. 1 illustrates an example of a configuration of a terminal apparatus according to the embodiment of the present invention;
FIG. 2 illustrates an example of a configuration of a server apparatus according to the embodiment of the present invention;
FIG. 3 illustrates an example of an identification information management table according to the embodiment of the present invention;
FIG. 4 illustrates an example of an UI information management table according to the embodiment of the present invention;
FIG. 5 illustrates an example of an operation of the terminal apparatus and the server apparatus according to the embodiment of the present invention;
FIG. 6A to FIG. 6C illustrate display screens of the terminal apparatus according to the embodiment of the present invention;
FIG. 7 illustrates an example of an operation of the terminal apparatus and the server apparatus according to the embodiment of the present invention; and
FIG. 8 illustrates an example of the display screen of the terminal apparatus according to the embodiment of the present invention.

### Description of Embodiments

An embodiment of the present invention is described below in detail with reference to the accompanying drawings. It is to be noted that the embodiment described below is merely an example, and the present invention is not limited to the embodiment.

### <Configuration of terminal apparatus>

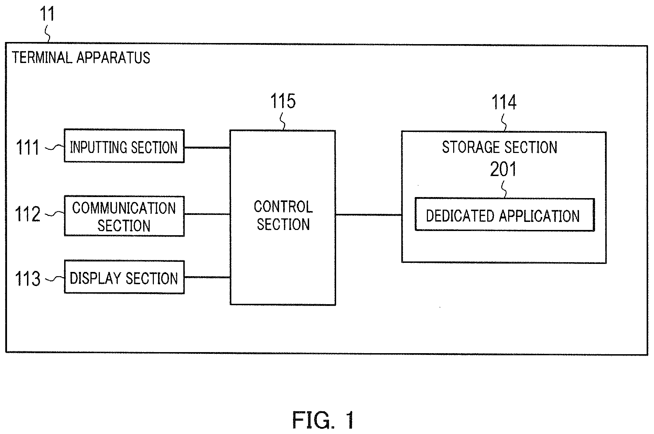

First, a configuration of terminal apparatus 11 according to the embodiment of the present invention is described with reference to FIG. 1. FIG. 1 illustrates an example of the configuration of terminal apparatus 11 according to the present embodiment.

Terminal apparatus 11 is an apparatus (such as a personal computer, a smartphone, a tablet or the like, for example) that is used by a patient in a pharmacy. A patient can use the service provided through dedicated application 201 in terminal apparatus 11.

Examples of the service include a function that allows the patient to browse his or her medicine-taking history, a function that allows the patient to preliminarily transmit, to a pharmacy, data on a medical prescription provided from a hospital, a function that allows the patient to confirm the setting of dedicated application 201, a function that allows the patient to confirm the given points corresponding to the use of a pharmacy, a function that allows the patient to confirm the medical expenses paid to a pharmacy, a function that allows the patient to browse a member site (member's website) of a pharmacy, a function that allows the patient to browse an EC site of a pharmacy to purchase a medicinal product and the like, a function that allows the patient to fill a questionnaire of a pharmacy, and the like.

As illustrated in FIG. 1, terminal apparatus 11 includes inputting section 111, communication section 112, display section 113, storage section 114, and control section 115.

Inputting section 111 is an inputting apparatus such as a touch panel, a keyboard, and a mouse. Inputting section 111 receives a patient's operation, and outputs the details of the received operation to control section 115.

For example, inputting section 111 receives an operation of requesting the activation of dedicated application 201 stored in storage section 114, an operation of inputting predetermined information, an operation of selecting a desired service and the like.

Communication section 112 is a communication apparatus including an antenna, a transmission circuit, a reception circuit and the like. Under the control of control section 115, communication section 112 exchanges information with server apparatus 12 through a predetermined network. The predetermined network may be a wired network, a wireless network, or a combination of a wired network and a wireless network.

For example, communication section 112 transmits information input by a patient to server apparatus 12 (which will be described in detail later).

In addition, for example, communication section 112 receives information sent from server apparatus 12 (which will be described in detail later).

Display section 113 is a display apparatus such as a display. Under the control of control section 115, display section 113 displays information.

For example, display section 113 displays the user interface (a display screen which can receive various kinds of operations of the user, which will be described in detail later) of dedicated application 201, service information provided by dedicated application 201, and the like.

Examples of the service information include a patient's medicine-taking history, a medical prescription of a patient received from a hospital, various kinds of settings of dedicated application 201, given points corresponding to the use of a pharmacy, patient's medical expenses paid to a pharmacy, a pharmacy's member site (member's website), a pharmacy's EC site, a questionnaire of a pharmacy, and the like.

Storage section 114 is a storage device such as a memory or a hard disk apparatus, and stores information.

For example, storage section 114 stores information received by communication section 112 from server apparatus 12 (which will be described in detail later).

In addition, for example, storage section 114 stores dedicated application 201. Dedicated application 201 is application software that operates on terminal apparatus 11, and provides the above-described services to patients. In the present embodiment, services are provided to patients in pharmacies with one dedicated application 201.

In dedicated application 201, a service common to pharmacies (hereinafter referred to as common service), or both a common service and a service of a specific pharmacy for making a differentiation from other pharmacies (hereinafter referred to as unique service) are provided.

Whether only a common service, or both a common service and a unique service is preliminarily set for each pharmacy. For example, in the case where the operating company of the dedicated application sets provision of a common service as free of charge, and provision of a unique service as payable, a pharmacy that has paid a predetermined fee to the operating company of the dedicated application can provide both the common service and the unique service in dedicated application 201. On the other hand, a pharmacy that has not paid a predetermined fee to the operating company of the dedicated application can provide only the common service in dedicated application 201. In this manner, a pharmacy can determine whether only a common service, or both the common service and the unique service in accordance with the budget.

The common service is, for example, a function that allows the patient to browse his or her medicine-taking history, a function that allows the patient to preliminarily transmit, to a pharmacy, data on a medical prescription provided from a hospital, a function that allows the patient to confirm the setting of dedicated application 201, and the like.

The unique service is, for example, a function that allows the patient to confirm the given points corresponding to the use of a pharmacy, a function that allows the patient to confirm the medical expenses paid to a pharmacy, a function that allows the patient to browse a member site (member's website) of a pharmacy, a function that allows the patient to browse an EC site of a pharmacy to purchase a medicinal product and the like, a function that allows the patient to fill a questionnaire of a pharmacy, and the like.

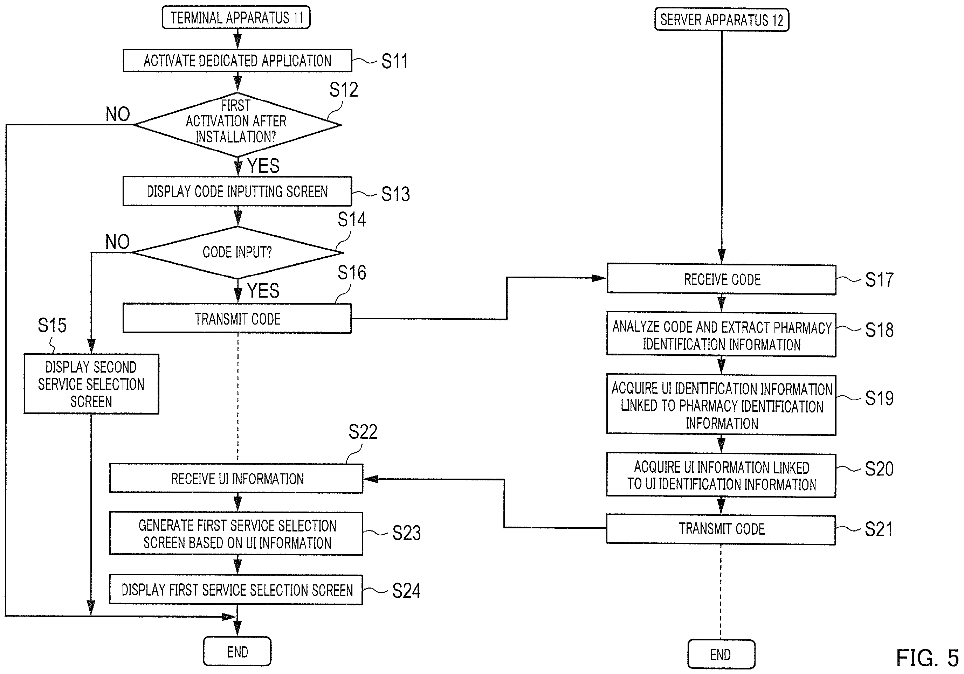

Control section 115 is a control device such as a processor, and controls processes of terminal apparatus 11. The control of control section 115 is described in detail later with reference to FIG. 5 to FIG. 8.

### <Configuration of server apparatus>

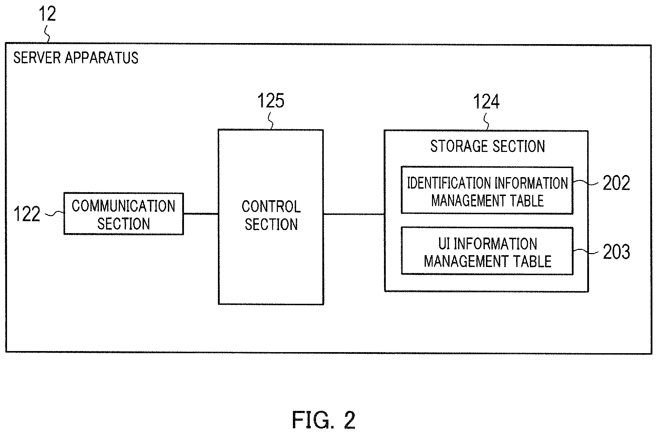

Next, a configuration of server apparatus 12 according to the embodiment of the present invention is described with reference to FIG. 2. FIG. 2 illustrates an example of the configuration of server apparatus 12 according to the present embodiment.

Server apparatus 12 is, for example, an apparatus that is installed and managed by the business (the operating company of the dedicated application) that manages dedicated application 201.

As illustrated in FIG. 2, server apparatus 12 includes communication section 122, storage section 124, and control section 125.

Communication section 122 is a communication apparatus including a transmission circuit, a reception circuit and the like. Under the control of control section 125, communication section 122 exchanges information with terminal apparatus 11 through a predetermined network.

For example, communication section 122 receives information sent from terminal apparatus 11 (which will be described in detail later).

In addition, for example, communication section 122 transmits information acquired based on information received from terminal apparatus 11 (which will be described in detail later), to terminal apparatus 11.

Storage section 124 is a storage device such as a memory or a hard disk apparatus, and stores information.

For example, storage section 124 stores information received by communication section 122 from terminal apparatus 11 (which will be described in detail later).

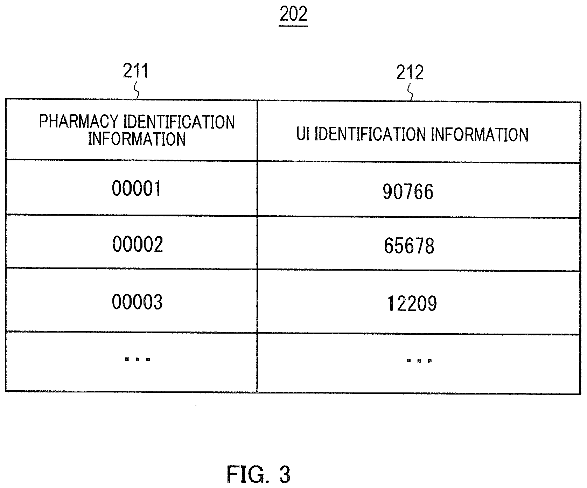

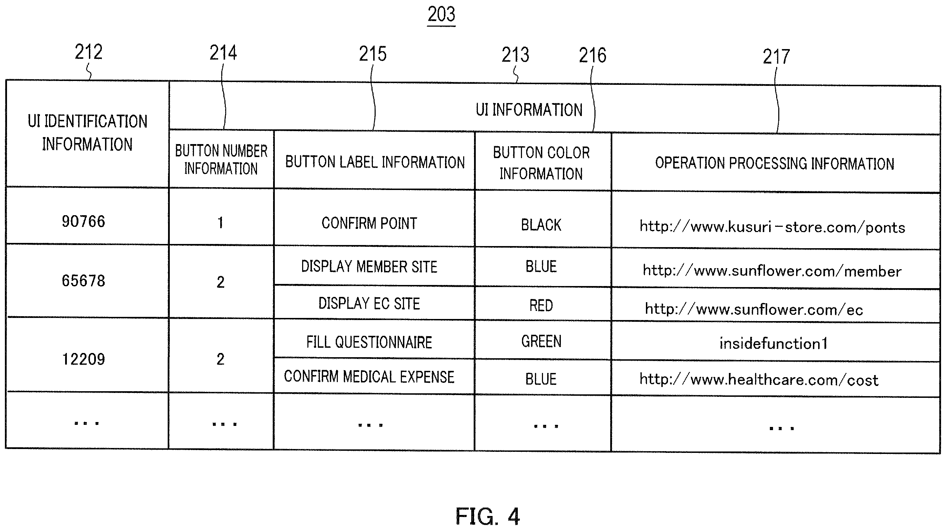

In addition, for example, storage section 124 stores identification information management table 202, and UI (User Interface) information management table 203. The details of identification information management table 202 are described later with reference to FIG. 3, and the details of UI information management table 203 are described later with reference to FIG. 4.

Control section 125 is a control device such as a processor, and controls processes of server apparatus 12. The details of the control of control section 125 are described later with reference to FIG. 5 to FIG. 8.

### <Identification information management table>

Next, identification information management table 202 is described with reference to FIG. 3. FIG. 3 illustrates an example of identification information management table 202 stored in storage section 124 of server apparatus 12.

As illustrated in FIG. 3, in identification information management table 202, pharmacy identification information 211 and UI identification information 212 are linked to each other.

Pharmacy identification information 211 is identification information of a pharmacy.

UI identification information 212 is identification information of a first service selection screen of dedicated application 201. The first service selection screen is an user interface that includes options of the common service (for example, buttons 306 to 308 described later) and the options of the unique service (for example, buttons 309 and 310) described later, and receives an operation of a patient of selecting a desired service from among the options of the common service and the options of the unique service.

### <UI information management table>

Next, UI information management table 203 is described with reference to FIG. 4. FIG. 4 illustrates an example of UI information management table 203 stored in storage section 124 of server apparatus 12.

As illustrated in FIG. 4, in UI information management table 203 (an example of the screen information management table), UI identification information 212 and UI information 213 are linked to each other.

As described above, UI identification information 212 (an example of the screen identification information) is identification information of the first service selection screen of dedicated application 201.

UI information 213 (an example of the screen information) is information on the details of the first service selection screen. UI information 213 is used for generating the first service selection screen in terminal apparatus 11.

As illustrated in FIG. 4, UI information 213 includes button number information 214, button label information 215, button color information 216, and operation processing information 217.

Button number information 214 is information representing the number of buttons included in the first service selection screen. The buttons are buttons for receiving an operation of the patient of selecting a unique service.

Button label information 215 is information representing a label displayed on the button. The label is, for example, a character string indicating a unique service. Button label information 215 is registered in accordance with the number of buttons indicated by button number information 214.

Button color information 216 is information representing the colors of the buttons. Button color information 216 is registered for each label represented by button label information 215.

Operation processing information 217 is information representing a process of a unique service which should be executed in response to a button operation. Operation processing information 217 is registered for each label represented by button label information 215.

It is to be noted that UI information 213 may contain information other than the information illustrated in FIG. 4. For example, information representing the background color other than the buttons (for example, the brand color of a pharmacy and the like), image information representing a logo of a pharmacy and the like may be included.

In addition, for example, the information included in UI information 213 may be updated by an inputting operation of a worker of the operating company of the dedicated application, or may be updated by receiving information from an external apparatus not illustrated.

### <Example operation 1 of terminal apparatus and server apparatus>

Next, example operation 1 (an example of the display control method) of server apparatus 12 and terminal apparatus 11 according to the embodiment of the present invention is described with reference to FIG. 5. FIG. 5 illustrates example operation 1 of terminal apparatus 11 and server apparatus 12.

First, when inputting section 111 receives an operation of requesting activation of dedicated application 201, control section 115 of terminal apparatus 11 reads dedicated application 201 from storage section 114, and activates dedicated application 201 (step S11).

Next, control section 115 determines whether the activation of dedicated application 201 is the first activation after the installation (step S12).

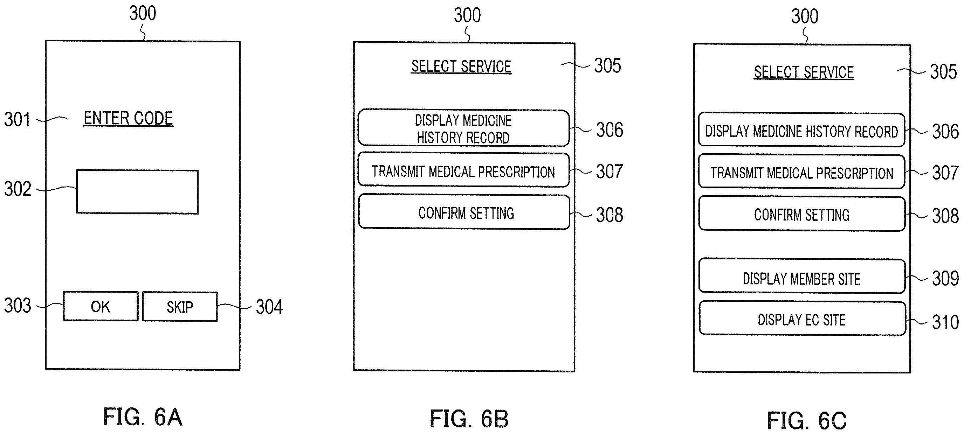

When it is determined at step S12 that the activation is not the first activation after the installation (step S12: NO), the series of processes are completed. It is to be noted that, when it is determined that the activation is not the first activation after the installation, the following operation may be performed. For example, in the case where the first service selection screen (see FIG. 6C) is displayed at the last activation, control section 115 may control display section 113 to display the first service selection screen. In addition, for example, in the case where the second service selection screen (see FIG. 6B) is displayed at the last activation, control section 115 may control display section 113 to display the second service selection screen.

When it is determined at step S12 that the activation is the first activation after the installation (step S12: YES), control section 115 controls display section 113 to display a code inputting screen. With this control, display section 113 displays a code inputting screen (step S13).

The code inputting screen is a user interface for receiving an operation of inputting a code. A code is, for example, a character string (such as numbers, alphabet, marks, or, combinations thereof, for example) containing pharmacy identification information 211. In the present embodiment, a code is composed of 5-digit number, and the 5-digit number is used as pharmacy identification information 211, for example.

Here, an example of the code inputting screen is described with reference to FIG. 6A. FIG. 6A illustrates an example of the code inputting screen. Here, a touch panel is described as an example.

As illustrated in FIG. 6A, in screen 300 of display section 113, message 301 for facilitating input of a code, entry field 302 of a code, button 303 for receiving an operation of requesting completion of input of a code, and button 304 for receiving an operation of requesting skip of input of a code are displayed.

In the code inputting screen illustrated in FIG. 6A, a patient inputs in entry field 302 a code received form workers of a pharmacy by word of mouth, or a code written in a flyer or the like received at a pharmacy for example. Then, when completing the input of the code, the patient presses down button 303. Alternatively, when skipping the input of a code, the patient presses down button 304 in the code inputting screen illustrated in FIG. 6A.

Now the description is returned to FIG. 5.

Control section 115 determines whether the code is input (step S14). For example, when a code is input in entry field 302 illustrated in FIG. 6A and button 303 is pressed down, control section 115 determines that the code is input. When the code is not input in entry field 302 illustrated in FIG. 6A and button 304 is pressed down, control section 115 determines that the code is not input.

When it is determined at step S14 that the code is not input (step S14: NO), control section 115 controls display section 113 to display the second service selection screen. With this control, display section 113 displays the second service selection screen (step S15).

The second service selection screen is a user interface that does not include the options (for example, buttons 309 and 310 described later) of the unique service, but includes the options (for example, buttons 306 to 308 described later) of the common service, and, receives an operation of a patient of selecting a desired service from among the options of the common service.

Here, an example of the second service selection screen is described with reference to FIG. 6B. FIG. 6B illustrates an example of the second service selection screen. Here, a touch panel is described as an example.

As illustrated in FIG. 6B, in screen 300 of display section 113, message 305 for facilitating selection of a service, button 306 for receiving an operation of selecting a medicine-taking history display service, button 307 for receiving an operation of selecting a medical prescription transmission service, and button 308 for receiving an operation of selecting a setting confirmation service are displayed.

The medicine-taking history display service is a function that allows the patient to browse his or her medicine-taking history. In addition, the medical prescription transmission service is a function that allows the patient to preliminarily transmit, to a pharmacy, data on a medical prescription provided from a hospital. In addition, the setting confirmation service is a function that allows the patient to confirm the setting of dedicated application 201. It is to be noted that, in the example illustrated in FIG. 6B, the common service is the medicine-taking history display service, the medical prescription transmission service, and the setting confirmation service as an example, the common service is not limited to this.

In the second service selection screen illustrated in FIG. 6B, the patient presses down any of buttons 306 to 308 displayed as the options of the common service to select the desired service. Then, control section 115 executes the service corresponding to the pressed button.

Now the description is returned to FIG. 5.

When it is determined at step S14 that a code is input (step S14: YES), control section 115 controls communication section 112 to transmit the code to server apparatus 12. With this control, communication section 112 transmits the code to server apparatus 12 (step S16).

Next, communication section 122 of server apparatus 12 receives the code from terminal apparatus 11 (step S17).

Next, control section 125 analyzes the code and extracts pharmacy identification information 211 (step S18).

Next, control section 125 reads identification information management table 202 from storage section 124, and acquires UI identification information 212 linked to pharmacy identification information 211 extracted from the code (step S19). For example, in the case where pharmacy identification information 211 extracted from the code is "00001," UI identification information 212 "90766" is acquired in identification information management table 202 illustrated in FIG. 3.

Next, UI information management table 203 is read from storage section 124, and UI information 213 linked to UI identification information 212 acquired from identification information management table 202 is acquired (step S20). For example, in the case where UI identification information 212 acquired from identification information management table 202 is "90766," button number information 214 "1," button label information 215 "point confirmation," button color information 216 "black," and operation processing information 217 "http://www.kusuri-store.com/ponts" are acquired in UI information management table 203 illustrated in FIG. 4.

Next, control section 125 controls communication section 122 to transmit UI information 213 acquired from UI information management table 203 to terminal apparatus 11. With this control, communication section 122 transmits UI information 213 to terminal apparatus 11 (step S21).

Next, communication section 112 of terminal apparatus 11 receives UI information 213 from server apparatus 12 (step S22).

Next, control section 115 generates the first service selection screen based on UI information 213 (step S23), and controls display section 113 to display the first service selection screen. With this control, display section 113 displays the first service selection screen (step S24).

As described above, the first service selection screen is a user interface for receiving an operation of a patient of selecting a desired service from among the common service and the unique service.

Here, an example of the first service selection screen is described with reference to FIG. 6C. FIG. 6C illustrates an example of the first service selection screen. Here, a touch panel is described as an example. In addition, the first service selection screen illustrated in FIG. 6C is generated based on UI information 213 linked to UI identification information 212 "65678" in UI information management table 203 illustrated in FIG. 4.

As illustrated in FIG. 6C, in screen 300 of display section 113, message 305 and buttons 306 to 308 are displayed as with the second service selection screen illustrated in FIG. 6B. Further, in screen 300 of display section 113, button 309 for receiving an operation of selecting the member site display service, and button 310 for receiving an operation of selecting the EC site display service are displayed. At this time, button 309 is displayed in blue, and a label "display member site" is displayed in button 309. In addition, button 310 is displayed in red, and a label "display EC site" is displayed in button 10.

The member site display service is a function that allows the patient to browse a member site (member's website) of a pharmacy. In addition, the EC site display service is a function that allows the patient to browse an EC site of a pharmacy to purchase a medicinal product and the like. It is to be noted that, in the example illustrated in FIG. 6C, the unique service is the member site display service and the EC site display service as an example, the unique service is not limited to this.

In the first service selection screen illustrated in FIG. 6C, the patient presses down any of buttons 306 to 308 displayed as the options of the common service and button 309 to 310 displayed as the options of the unique service to select a desired service. Then, control section 115 executes the service corresponding to the pressed button. For example, in the case where button 309 is pressed down, control section 115 accesses the URL "http://www.sunflower.com/member" represented by operation processing information 217. In addition, for example, in the case where button 310 is pressed down, control section 115 accesses the URL "http://www.sunflower.com/ec" represented by operation processing information 217.

It is to be noted that, as described above, in the case where information representing a pharmacy's brand color, image information representing a pharmacy's logo and/or the like are contained as UI information 213 for example, the background other than buttons 306 to 310 may be displayed in the brand color, and the pharmacy's logo may be displayed at a predetermined position in the first service selection screen illustrated in FIG. 6C.

### <Example operation 2 of terminal apparatus and server apparatus>

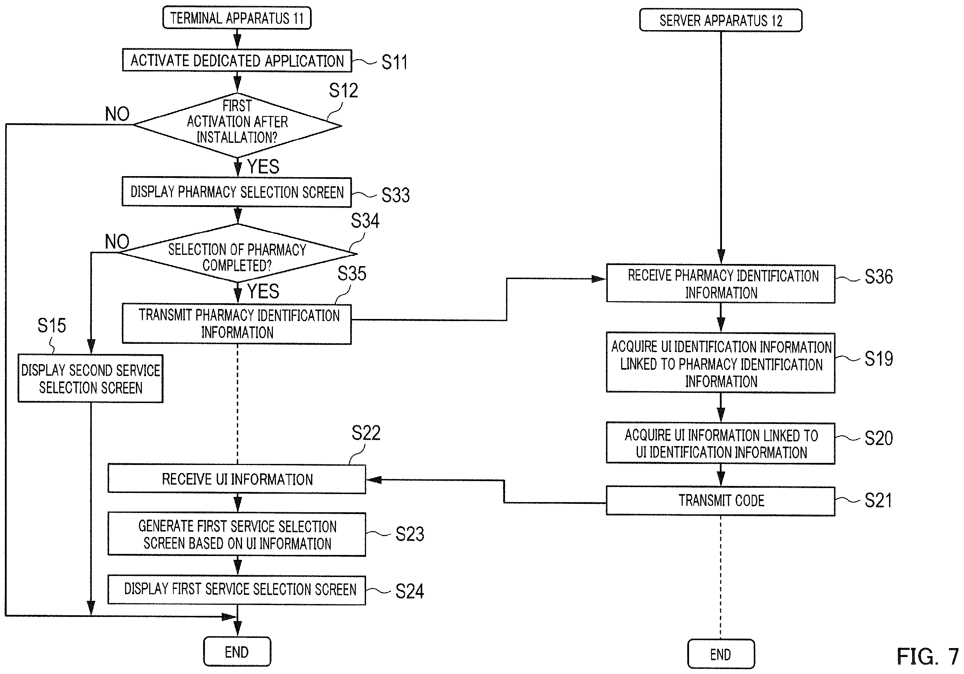

Next, example operation 2 (an example of the display control method) of server apparatus 12 and terminal apparatus 11 according to the embodiment of the present invention is described with reference to FIG. 7. FIG. 7 illustrates example operation 2 of terminal apparatus 11 and server apparatus 12. In FIG. 7, the steps identical to those of FIG. 5 are denoted with the same reference numerals. In the following, the description of the steps identical to those of FIG. 5 is omitted, and steps S33 to S36, which are different from FIG. 5, are described.

Control section 115 determines whether the activation of dedicated application 201 is the first activation after the installation (step S12), and when the activation is the first activation after the installation (step S12: YES), control section 115 controls display section 113 to display a pharmacy selection screen. With this control, display section 113 displays the pharmacy selection screen (step S33).

The pharmacy selection screen is a user interface for receiving an operation of selecting pharmacies.

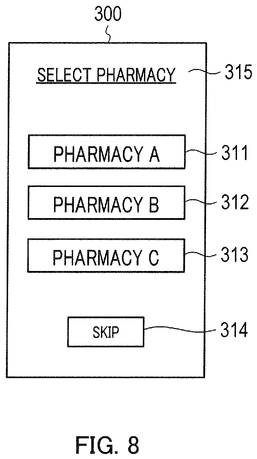

Here, an example of the pharmacy selection screen is described with reference to FIG. 8. FIG. 8 illustrates an example of the pharmacy selection screen. Here, a touch panel is described as an example.

As illustrated in FIG. 8, in screen 300 of display section 113, message 315 for facilitating selection of pharmacies, button 311 for receiving selection of pharmacy A, button 312 for receiving selection of pharmacy B, button 313 for receiving selection of pharmacy C, and button 314 for receiving an operation of requesting skip of selection of pharmacies are displayed. It is to be noted that, while the options of the pharmacy are buttons 311 to 313 in FIG. 8, the number of the options is not limited to this.

On the pharmacy selection screen illustrated in FIG. 8, the patient presses down any of buttons 311 to 313 corresponding to the desired pharmacy, for example. Alternatively, when skipping selection of pharmacies, the patient presses down button 314 on the pharmacy selection screen illustrated in FIG. 8.

Now the description is returned to FIG. 7.

Control section 115 determines whether a pharmacy is selected (step S34). For example, when any of buttons 311 to 313 illustrated in FIG. 8 is pressed down, control section 115 determines that a pharmacy is selected. When buttons 311 to 313 illustrated in FIG. 8 are not pressed down but button 314 is pressed down, control section 115 determines that a pharmacy is not selected.

When it is determined at step S34 that a pharmacy is not input (step S34: NO), control section 115 controls display section 113 to display the second service selection screen. With this control, display section 113 displays the second service selection screen (step S15). As described above, the second service selection screen is a user interface for receiving an operation of a patient of selecting a desired service from the common service (see FIG. 6B).

When it is determined at step S34 that a pharmacy is selected (step S34: YES), control section 115 performs the following process. In the present example operation, a table in which buttons 311 to 313 and pharmacy identification information 211 are linked to each other is stored in storage section 114 of terminal apparatus 11, for example. Control section 115 reads the table from storage section 114, and acquires pharmacy identification information 211 linked to the pressed button. Then, control section 115 controls communication section 112 to transmit the acquired pharmacy identification information 211 to server apparatus 12. With this control, communication section 112 transmits pharmacy identification information 211 to server apparatus 12 (step S35).

Next, communication section 122 of server apparatus 12 receives pharmacy identification information 211 from terminal apparatus 11 (step S36).

Next, control section 125 reads identification information management table 202 from storage section 124, and acquires UI identification information 212 linked to the received pharmacy identification information 211 (step S19).

### <Operation and effect of embodiment

As described above, the present embodiment brings about the following operation and effect.

According to the present embodiment, when input of a code or selection of pharmacies is not performed, only the options of the common service are displayed, whereas when input of a code or selection of pharmacies is performed, the options of the common service and the options of the unique service are displayed. With this configuration, in the case where services are provided to the patients of pharmacies with one dedicated application, major pharmacies having sufficient funding can provide a unique service in addition the common service, for example. Small-to-medium pharmacies which do not have sufficient funding, on the other hand, can reduce the cost of developing and running a dedicated application and can request the patients to use the dedicated application, thus leading more patients to that pharmacy, for example. In addition, both the major pharmacies and small-to-medium pharmacies can make the patients to continuously use the same pharmacies by requesting the patients to use the dedicated application.

### <Modification of embodiment

While the embodiment of the present invention has been described above, the present invention should not be limited to the above-mentioned embodiment, and various modifications may be made. Modifications are described below.

In the present embodiment, it is possible to adopt a configuration in which, when the patient selects a function (an example of the unique service) of browsing the information on a store of a pharmacy (information on business hours, location, price of the products, and the like) on the first service selection screen, only information on the stores of the pharmacies belonging to the same group (same parent company) is displayed, for example. With this configuration, the pharmacies that provide the unique service can prevent store information of other pharmacies from being presented to the patient, and it is thus possible to set dedicated application 201 appearing to be the unique application of that pharmacy.

In addition, while identification information management table 202 and UI information management table 203 are stored in storage section 124 of server apparatus 12 in the present embodiment, the present invention is not limited to this. For example, identification information management table 202 and UI information management table 203 may be stored in a storage section of an external apparatus (for example, a database server) installed separately from server apparatus 12. In this case, server apparatus 12 communicates with the external apparatus to read identification information management table 202 and UI information management table 203.

In addition, while the code input to terminal apparatus 11 by the patient is pharmacy identification information 211 in the present embodiment, the present invention is not limited to this. For example, the code may be pharmacy group identification information capable of identifying the group where the pharmacy belongs. As with pharmacy identification information 211, the pharmacy group identification information is composed of a character string. An example operation in this case is described below.

Pharmacy identification information of the pharmacy belonging to the group represented by the pharmacy group identification information, and pharmacy name information representing the name of the pharmacy (store) are stored in association with each other in storage section 124 of server apparatus 12 for each pharmacy group identification information.

First, when the patient inputs a code including pharmacy group identification information on the code inputting screen, terminal apparatus 11 transmits the code to server apparatus 12.

Next, server apparatus 12 analyzes the code received from terminal apparatus 11 to extract the pharmacy group identification information, reads the pharmacy identification information and the pharmacy name information associated with the pharmacy group identification information from storage section 124, and transmits the information to terminal apparatus 11.

Next, terminal apparatus 11 controls display section 113 to display the pharmacy selection screen on which the pharmacy name information received from server apparatus 12 can be selected. In this manner, a pharmacy selection screen on which a multiple pieces of pharmacy name information are listed is displayed on display section 113, for example.

Next, when the patient selects the pharmacy name information, terminal apparatus 11 transmits the pharmacy identification information corresponding to the pharmacy name information to server apparatus 12. The subsequent operations are identical to the operations subsequent to step S19 of FIG. 5 and FIG. 7.

In this manner, according to the present example operation, the code input by the patient is the pharmacy group identification information, and thus the pharmacy can cause the patient to select the pharmacies of the own group, and thus can make the patients to continuously use the same pharmacies.

In addition, the functions of terminal apparatus 11 and server apparatus 12 described in the embodiment may be achieved with a computer program. To be more specific, when a CPU (Central Processing Unit) in a computer copies a program stored in a storage apparatus into a RAM (Random Access Memory), and sequentially reads and executes an order included in the program, the above-described functions are achieved.

This application is entitled to and claims the benefit of Japanese Patent Application No. 2015-101912 dated May 19, 2015, the disclosure of which including the specification, drawings and abstract is incorporated herein by reference in its entirety.

### Industrial Applicability

The present invention is applicable to a terminal apparatus for controlling a display screen of application software, a server apparatus, a display control method, and, a display control program.

### Reference Signs List

11 Terminal apparatus
12 Server apparatus
111 Inputting section
112, 122 Communication section
113 Display section
114, 124 Storage section
115, 125 Control section
201 Dedicated application
202 Identification information management table
203 UI information management table
211 Pharmacy identification information
212 UI identification information
213 UI information
214 Button number information
215 Button label information
216 Button color information
217 Operation processing information

## Claims

1. A terminal apparatus for operating an application for providing a service of a plurality of pharmacies, the terminal apparatus comprising:
a communication section that exchanges information with a server apparatus;
a display section that displays the application; and
a control section that controls the communication section and the display section, wherein
when an operation of identifying a pharmacy is received in the application, the control section controls the communication section to transmit information of the operation to the server apparatus,
when the communication section receives screen information corresponding to a pharmacy identified by the server apparatus based on the information of the operation from the server apparatus, the control section generates a first service selection screen based on the screen information, and controls the display section to display the first service selection screen,
when the operation is not received in the application, the control section controls a display section to display a second service selection screen,
the first service selection screen is a screen that includes options of a common service common to the plurality of pharmacies, and options of a unique service unique to the pharmacy identified by the server apparatus, and receives an operation of a user of the terminal apparatus for selecting a desired service from among the options of the common service and the options of the unique service, and
the second service selection screen is a screen that includes the options of the common service without including the options of the unique service, and receives an operation of the user of the terminal apparatus for selecting a desired service from among the options of the common service.

2. The terminal apparatus according to claim 1, wherein:
the control section controls the display section to display a code inputting screen for receiving an operation of inputting a code used for identification of the pharmacy;
when a predetermined code is input on the code inputting screen, the control section controls the communication section to transmit to the code to the server apparatus; and
the communication section receives from the server apparatus screen information corresponding to a pharmacy identified by the server apparatus based on the code.

3. The terminal apparatus according to claim 2, wherein when the application is activated for a first time after installation of the application, the control section controls the display section to display the code inputting screen.

4. The terminal apparatus according to claim 1, wherein:
the control section controls the display section to display a pharmacy selection screen for receiving an operation of selecting a pharmacy;
when a specific pharmacy is selected on the pharmacy selection screen, the control section controls the communication section to transmit pharmacy identification information capable of identifying the pharmacy to the server apparatus; and
the communication section receives screen information corresponding to a pharmacy identified by the server apparatus based on the pharmacy identification information from the server apparatus.

5. The terminal apparatus according to claim 1, wherein:
the server apparatus stores pharmacy identification information capable of identifying the pharmacy and pharmacy name information representing a name of the pharmacy in association with each other for each pharmacy group identification information capable of identifying a group where the pharmacy belongs;
the control section controls the display section to display a code inputting screen for receiving an operation of inputting a code including the pharmacy group identification information;
when a predetermined code is input on the code inputting screen, the control section controls the communication section to transmit to the code to the server apparatus,
the communication section receives, from the server apparatus, the pharmacy name information and the pharmacy identification information identified based on the pharmacy group identification information obtained by analysis of the code in the server apparatus,
the control section controls the display section to display a pharmacy selection screen for receiving an operation of selecting a pharmacy represented by the pharmacy name information; and
when a specific pharmacy is selected on the pharmacy selection screen, the control section controls the communication section to transmit the pharmacy identification information corresponding to the pharmacy to the server apparatus.

6. The terminal apparatus according to claim 4 or 5, wherein when the application is activated for a first time after installation of the application, the control section controls the display section to display the pharmacy selection screen.

7. A server apparatus comprising:
a communication section that exchanges information with a terminal apparatus, the terminal apparatus being configured to operate an application for providing a service of a plurality of pharmacies, and display a first service selection screen, the first service selection screen being configured to include options of a common service common to the plurality of pharmacies and options of a unique service unique to a pharmacy, and receive an operation of a user of the terminal apparatus for selecting a desired service from among the options of the common service and the options of the unique service;
a storage section that stores an identification information management table in which screen identification information capable of identifying screen information of the application is linked for each pharmacy, and a screen information management table in which the screen information is linked for each screen identification information; and
a control section that acquires, when an operation of identifying a pharmacy is received in the application of the terminal apparatus and information of the operation is received from the terminal apparatus, the screen identification information from the identification information management table based on the information of the operation, acquires the screen information from the screen information management table based on the screen identification information, and controls the communication section to transmit the screen information to the terminal apparatus, wherein
in the terminal apparatus, the screen information is used for generating a second service selection screen, the second service selection screen being configured to include options of a common service common to the plurality of pharmacies and options of a unique service unique to a pharmacy identified by the operation, and receive an operation of the user of the terminal apparatus for selecting a desired service from among the options of the common service and the options of the unique service.

8. A display control method of a terminal apparatus for operating an application for providing a service of a plurality of pharmacies, the method comprising:
when an operation of identifying a pharmacy is received in the application, generating a first service selection screen based on screen information corresponding to a pharmacy identified based on information of the operation, and controlling a display section to display the first service selection screen; and
when the operation is not received in the application, controlling the display section to display a second service selection screen, wherein
the first service selection screen is a screen that includes options of a common service common to the plurality of pharmacies, and options of a unique service unique to a pharmacy identified by a server apparatus, and receives an operation of a user of the terminal apparatus for selecting a desired service from among the options of the common service and the options of the unique service, and
the second service selection screen is a screen that includes the options of the common service without including the options of the unique service, and receives an operation of the user of the terminal apparatus for selecting a desired service from among the options of the common service.

9. A display control program to be executed by a computer for operating an application for providing a service of a plurality of pharmacies, wherein:
the program causes the computer to, when an operation of identifying a pharmacy is received in the application, generate a first service selection screen based on screen information corresponding to a pharmacy identified based on information of the operation, and control the display section to display the first service selection screen;
the program causes the computer to, when the operation is not received in the application, control the display section to display a second service selection screen;
the first service selection screen is a screen that includes options of a common service common to the plurality of pharmacies, and options of a unique service unique to a pharmacy identified by a server apparatus, and receives an operation of a user of the terminal apparatus for selecting a desired service from among the options of the common service and the options of the unique service; and
the second service selection screen is a screen that includes the options of the common service without including the options of the unique service, and receives an operation of the user of the terminal apparatus for selecting a desired service from among the options of the common service.
